# EUROPEAN PATENT APPLICATION

(11) **EP 4 804 194 A1**
(43) Date of publication of application: **09.09.2026**
(21) Application number: 25161567.0
(22) Date of filing: 04.03.2025
(51) Int. Cl.: G16B 20/00, G16B 25/10, G16B 40/20

(54) **TARGET IDENTIFICATION**

(71) Applicant: Bayer Aktiengesellschaft, 51373 Leverkusen (DE)
(72) Inventor: SCHNECKENER, Sebastian, 51373 Leverkusen (DE); MRUGALLA, Florian, 51373 Leverkusen (DE); BITTON, Adrien David Pierre, 51373 Leverkusen (DE); PEYRARD, Stephane, 69009 Lyon (DE); LEGGIO, Bruno, 69009 Lyon (DE); DI VIETRO, Luigi, 69009 Lyon (FR)
(74) Representative: BIP Patents

(57) **Abstract**

Systems, methods, and computer programs disclosed herein relate to the identification of target genes and/or gene products in pest organisms.

## Description

### FIELD OF THE DISCLOSURE

Systems, methods, and computer programs disclosed herein relate to the identification of target genes and/or gene products in pest organisms.

### BACKGROUND

A target gene or a gene product (e.g., a protein) within a pest organism is a specific gene or gene product, which plays a critical role in the organism's survival, reproduction, and/or ability to cause damage to crops. A target gene or gene product is one that is essential for the pest's life processes such as feeding, growth, development, and/or reproduction. These targets are integral to the pest's ability to thrive and, importantly, to its interactions with the crops it infests. The proteins encoded by these genes may be involved in key biological pathways, structural functions, and/or in the pest's defense mechanisms against plant defense compounds.

The identification of target genes or gene products in pest organisms represents a cutting-edge frontier in the development of crop protection products. By identifying and understanding these targets, scientists can devise highly specific and efficient strategies to protect crops from pests, thereby enhancing food security and agricultural sustainability.

The identification of target genes or gene products typically involves several techniques that span molecular biology, chemistry, biochemistry, genetics, and bioinformatics. Initially, comparative genomics and transcriptomics can uncover genes that are uniquely expressed in the pest or are significantly upregulated during host-pest interactions. Functional assays, such as gene knockdown or knockout experiments using RNA interference (RNAi) or gene editing, can then verify the role of these genes in the pest's biology. Proteomics and metabolomics provide further insights into the activity of the proteins encoded by these genes and their place within essential metabolic or signalling pathways.

As laboratory methods are often complicated, costly and time-consuming, computational approaches have been developed to identify so-called essential genes and/or gene products (e.g., proteins). An essential gene or gene product refers to a gene or its product that is necessary for the survival of an organism. These genes or gene products are involved in critical biological processes that are fundamental to the organism's life cycle, such as replication, transcription, translation, cell structure maintenance, and/or metabolism. If an essential gene is disrupted or if its product is inhibited or rendered nonfunctional, it can lead to the death of the cell or organism or cause severe developmental or physiological defects. An essential gene or gene product is therefore a potential target for new crop protection products.

In a comprehensive review article, advanced computational methods for identifying essential genes or proteins within biological networks are discussed, emphasizing the importance of these components in understanding complex diseases, minimal genome requirements for cell life, and drug target development (X. Li et al.: Network-based methods for predicting essential genes or proteins: a survey, Briefings in Bioinformatics, 2020, vol. 21, issue 2). The review article categorizes the methods into four main approaches: topology-based methods, integration of protein-protein interaction (PPI) networks with biological data, dynamic network-based methods, and machine learning approaches.

The identification of essential genes and/or gene products as potential targets for new crop protection products is a promising approach that can still be improved.

### SUMMARY

In a first aspect, the present disclosure provides a computer-implemented method, the method comprising:
- determining a candidate, wherein the candidate is related to a gene and a gene product of a pest organism,
- generating a gene representation, wherein the gene representation is a numerical representation of the gene,
- generating a product representation, wherein the product representation is a numerical representation of the gene product,
- generating an interrelation representation, wherein the interrelation representation is a numerical representation of interrelations of the gene and/or gene product with other genes and/or gene products of the organism,
- generating a gene expression response representation, wherein the gene expression response representation is a numerical representation of one or more responses of an expression level of the gene to one or more external stimuli,
- generating a candidate representation based on the gene representation, the product representation, the interrelation representation, and the gene expression response representation,
- providing a trained machine learning model, wherein the machine learning model is configured and was trained to determine how essential a gene and/or gene product is for an organism,
- inputting the candidate representation into the trained machine learning model,
- receiving information about how essential the gene and/or gene product is to the organism as an output from the trained machine learning model,
- outputting the information.

In another aspect, the present disclosure provides a computer system comprising:
a processing unit; and
a memory storing a computer program configured, when executed by the processing unit, to cause the computer system to perform the following:
   - determining a candidate, wherein the candidate is related to a gene and a gene product of a pest organism,
   - generating a gene representation, wherein the gene representation is a numerical representation of the gene,
   - generating a product representation, wherein the product representation is a numerical representation of the gene product,
   - generating an interrelation representation, wherein the interrelation representation is a numerical representation of interrelations of the gene and/or gene product with other genes and/or gene products of the organism,
   - generating a gene expression response representation, wherein the gene expression response representation is a numerical representation of one or more responses of an expression level of the gene to one or more external stimuli,
   - generating a candidate representation based on the gene representation, the product representation, the interrelation representation, and the gene expression response representation,
   - providing a trained machine learning model, wherein the machine learning model is configured and was trained to determine how essential a gene and/or gene product is for an organism,
   - inputting the candidate representation into the trained machine learning model,
   - receiving information about how essential the gene and/or gene product is to the organism as an output from the trained machine learning model,
   - outputting the information.

In another aspect, the present disclosure provides a non-transitory computer readable storage medium having stored thereon a computer program that, when executed by a processing unit of a computer system, cause the computer system to execute the following steps:
- determining a candidate, wherein the candidate is related to a gene and a gene product of a pest organism,
- generating a gene representation, wherein the gene representation is a numerical representation of the gene,
- generating a product representation, wherein the product representation is a numerical representation of the gene product,
- generating an interrelation representation, wherein the interrelation representation is a numerical representation of interrelations of the gene and/or gene product with other genes and/or gene products of the organism,
- generating a gene expression response representation, wherein the gene expression response representation is a numerical representation of one or more responses of an expression level of the gene to one or more external stimuli,
- generating a candidate representation based on the gene representation, the product representation, the interrelation representation, and the gene expression response representation,
- providing a trained machine learning model, wherein the machine learning model is configured and was trained to determine how essential a gene and/or gene product is for an organism,
- inputting the candidate representation into the trained machine learning model,
- receiving information about how essential the gene and/or gene product is to the organism as an output from the trained machine learning model,
- outputting the information.

### BRIEF DESCRIPTION OF THE DRAWINGS

Fig. 1 shows an example of how to train the machine learning model of the present disclosure.
Fig. 2 shows an example of the use of a trained machine learning model of the present disclosure for prediction.
Fig. 3 shows an embodiment of the computer-implemented method in the form of a flowchart.
Fig. 4 illustrates a computer system according to some example implementations of the present disclosure in more detail.

### DETAILED DESCRIPTION

Various example embodiments will be more particularly elucidated below without distinguishing between the aspects of the disclosure (method, computer system, computer-readable storage medium). On the contrary, the following elucidations are intended to apply analogously to all the aspects of the disclosure, irrespective of in which context (method, computer system, computer-readable storage medium) they occur.

If steps are stated in an order in the present description or in the claims, this does not necessarily mean that the disclosure is restricted to the stated order. On the contrary, it is conceivable that the steps can also be executed in a different order or else in parallel to one another, unless, for example one step builds upon another step, this requiring that the building step be executed subsequently (this being, however, clear in the individual case). The stated orders may thus be exemplary embodiments of the present disclosure.

As used herein, the articles "a" and "an" are intended to include one or more items and may be used interchangeably with "one or more" and "at least one". As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The terms used in this disclosure have the meaning that these terms have in the prior art, in particular in the prior art cited in this disclosure, unless otherwise indicated.

The present disclosure provides means by which it can be determined how essential a gene or gene product is for an organism.

The organism is a plant pest.

A "plant pest" is an organism that may occur during the cultivation of a crop and may damage the crop, negatively affect the crop's harvest and/or may compete with the crop for natural resources. Examples of such plant pests are weeds, animal pests (such as beetles, caterpillars and worms), fungi, and pathogens (e.g., bacteria and viruses). Even if viruses are not classified as organisms from a biological point of view, they are still be included under the term organism in this disclosure.

In an embodiment of the present disclosure, the organism is an animal plant pest.

The animal plant pest may be an insect, such as an aphid, beetle, caterpillar, thrip, and/or whitefly. Animal plant pests can damage plants by chewing on leaves, stems, flowers, and/or roots, sucking sap, and/or spreading diseases.

The animal plant pest may be a mite. Mites can cause damage by feeding on plant tissues. Spider mites are a common example, known for spinning fine webs on the infested parts of plants.

The animal plant pest may be a nematode. A nematode is a worm-like organism that lives in soil and can attack plant roots, causing root knot, stunting, and/or other growth issues.

The animal plant pest may be a mollusk. Mollusks such as snails and slugs are common garden pests that feed on a wide range of plant materials, often leaving irregular holes in leaves and stems.

In another embodiment of the present disclosure, the organism is a fungus. Fungal pathogens can significantly impact plant health. Examples include powdery mildew, rusts, and blights. They typically cause spots, rots, and other symptoms on leaves, stems, and/or fruits. Fungi may also produce mycotoxins that make crops unsuitable for consumption.

In another embodiment of the present disclosure, the organism is a bacterium. Certain bacteria can cause diseases in plants, such as fire blight and bacterial wilt. Bacterial pathogens can lead to leaf spots, wilts, and/or cankers.

In another embodiment of the present disclosure, the plant pest is a virus. Plant viruses can lead to a wide range of symptoms, including mosaic patterns on leaves, stunted growth, and/or yield loss. They are often spread by insect vectors.

In another embodiment of the present disclosure, the organism is a weed. Weeds compete with crops for nutrients, water, and/or light. Some also harbor pests and diseases, making them indirect pests of plants. Weeds may also make a crop unsuitable for consumption at harvest time by producing alkaloids (e.g. Datura stramonium, Senecio vulgaris).

Based on its essentiality, a gene and/or gene product can then be selected as a possible target for a new crop protection product. In other words, information on how essential a gene or gene product is for the organism can indicate whether the gene or gene product is a potential target for a new crop protection product. If a gene or a gene product (e.g., the protein encoded by the gene) is essential for an organism, this gene or the gene product e.g., the encoded protein) is a potential target for a new crop protection product.

In this disclosure, the term "candidate" is used. The candidate is a potential target for a new crop protection product. The present disclosure provides means for determining whether the candidate is suitable as a target for a new crop protection product.

The term "candidate" refers to a gene and/or a gene product (such as a protein encoded by the gene). The new crop protection product may, for example, attack the gene to prevent the gene product being produced in a cell of the organism. The new crop protection product may, for example, attack the gene product to prevent it from performing the functions it normally fulfills in a cell of the organism.

A "gene product" is any molecule that is produced as a result of gene expression. This term primarily refers to proteins, which are the most common and well-studied gene products, but it also encompasses various types of RNA molecules that are transcribed from DNA. Gene products play crucial roles in cellular functions and processes, including structural, enzymatic, regulatory, and signaling activities. Examples of gene products include proteins. Proteins are functional molecules that perform a wide range of tasks within cells, such as enzymes, receptors, and/or structural components. Another example of a gene product is a non-coding RNA. A non-coding RNA is an RNA molecule that is not translated into a protein but may have important regulatory functions, including ribosomal RNA (rRNA), transfer RNA (tRNA), microRNAs (miRNAs), and/or long non-coding RNAs (lncRNAs). Another example of a gene product is a small RNA. Small RNAs include various types of small regulatory RNAs, such as small interfering RNAs (siRNAs), which are involved in the RNA interference (RNAi) pathway and play roles in gene silencing. Another example of a gene product is a peptide; some genes encode small peptides that may have biological functions, such as signaling or regulatory roles, but are not classified as full-length proteins.

In an embodiment of the present disclosure the term "gene product" refers to the protein encoded by the gene.

In a first step, the candidate is determined. The candidate can, for example, be entered and/or selected by a user in the computer system of the present disclosure.

The determination and/or selection of the candidate can be done based on the gene or the gene product; if it is done based on the gene, the gene product may follow automatically and *vice versa.*

The identified candidate usually automatically defines the organism. It is also possible that in a first step an organism is identified and/or selected and in a second step the candidate (a gene and/or gene product of the organism) is identified and/or selected or in reverse order. It is also possible that, in addition to the organism, a cell and/or cell type is selected in which the gene is expressed and/or the gene product occurs.

A machine learning model is used to determine how essential the gene and/or gene product is for the candidate. Such a computational model usually requires numbers as input.

In a further step, a numerical representation of the candidate is generated.

A numerical representation may be or include a number or an arrangement of numbers. A numerical representation may be or include a vector, a matrix, a tensor or another arrangement of numbers.

The term "feature vector" is also often used for a numerical representation. However, it should be noted that the present disclosure is not limited to vectors as numerical representations.

The candidate representation is a numerical representation of the candidate that represents various features of the candidate.

Such features may be features of the gene and/or the gene product and/or the organism and/or an interaction of the gene and/or gene product with other genes and/or gene products and/or one or more responses of an expression level of the gene to one or more external stimuli.

In an embodiment of the present disclosure, different numerical representations are generated for different features, which are then combined in a joint candidate representation.

Such a numerical representation may represent the gene and/or the gene product. Such a numerical representation may represent the interrelation of the gene with other genes of the organism. Such a numerical representation may represent the interrelation of the gene product with other gene products and/or genes of the organism. Such a numerical representation may represent a response of a level of expression of the gene to an external stimulus.

A numerical representation that is generated is a gene representation. The gene representation is a numerical representation of the gene.

In an embodiment of the present disclosure, the gene representation represents the structure of the gene, i.e. the sequence of nucleotides.

The primary structure of a gene, its nucleotide sequence, can be directly represented as a string of characters (A, C, G, T/U).

The nucleotide sequence of DNA and/or RNA can be converted into a numerical form using one-hot encoding, for example. "One-hot encoding" is a method to quantify categorical data. In short, this method produces a vector with length equal to the number of categories in the data set. If a data point belongs to the i^{th} category, then components of this vector are assigned the value 0 except for the i^{th} component, which is assigned a value of 1. So, in a one-hot encoding each nucleotide be represented by a unit binary vector of length n, containing a single one and n-1 zeros (e.g., [1,0,0, ..., 0] for one nucleotide and [0, 1, 0, ..., 0] for another nucleotide). For example, A = [1, 0, 0, 0], T/U = [0, 1, 0, 0], C = [0, 0, 1, 0], G = [0, 0, 0, 1].

Another example of a gene representation are k-mer counts. A k-mer refers to all possible subsequences (of length k) from a sequence of nucleotides. Counting the occurrences of each k-mer within a gene's sequence provides a high-dimensional, but very informative representation of the gene.

The structure of a gene, including its exons, introns, and regulatory elements, can be represented as a graph. In this representation, different parts of the gene (e.g., exons, regulatory elements) are nodes, and the relationships between them (e.g., adjacency, regulatory influence) are edges.

Specific features of gene structure, such as the presence or absence of certain regulatory elements or splicing variants, can be encoded as binary (1 or 0) in a binary vector.

The positions of specific elements within a gene (e.g., start and end positions of exons, positions of regulatory motifs) can be encoded as numerical values in a vector.

Another example of a gene representation is a Gene Ontology (GO) annotation. Gene Ontology provides a structured vocabulary for describing gene and gene product attributes across species. GO annotations related to biological processes, cellular components, and molecular functions can be used as categorical or binary features.

Genes can be represented by their involvement in metabolic and/or signalling pathways. This can be binary (involved/not involved) or more detailed, considering the gene's role in the pathway.

The gene representation may be an embedding generated by a trained machine learning model. An embedding of a gene refers to the representation of a gene's features and/or its relationships with other biological entities (such as other genes, proteins, and/or metabolic pathways) as a continuous, high-dimensional vector.

This concept borrows from the idea of embeddings in machine learning and natural language processing, where complex items (like words or images) are represented in a way that captures their relationships or similarities in a dense vector space. For genes, embeddings aim to encapsulate genetic information, functional characteristics, interactions, and potentially even evolutionary relationships in a format that can be efficiently processed by computational models.

To generate a gene embedding, a first step is to identify the data that are to be captured by the embedding. This could be the information about genes that have already been mentioned, such as gene sequence data, gene expression profiles (i.e. levels of gene expression across different conditions, tissues, and/or time points), information on gene function, pathways, and/or interactions with other genes/proteins, phylogenetic data (i.e. evolutionary relationships between genes across species) and/or other/further gene data.

To train a machine learning model, training data must be collected in a further step. Training data usually is a large amount of gene data from reference genes.

In a further step such gene data from reference genes may be pre-processed to convert it into a format suitable for model training. For gene sequence data, this may involve encoding the nucleotide sequences (e.g., via one-hot encoding or k-mer counting). For gene expression data, normalization techniques may be applied to ensure comparability across samples and/or conditions. Functional annotations may be encoded using binary indicators for the presence or absence of specific Gene Ontology (GO) terms. Phylogenetic data may be represented through phylogenetic trees or distance matrices.

In a further step, a machine learning model is selected, and a training task is chosen. The machine learning model takes the gene data as input, processes the data, generates an embedding and attempts to solve the training task based on the embedding. During training, the model learns to extract those features of the gene data and summarize them in the embedding that are relevant for solving the training task.

For example, the machine learning model may be or include an autoencoder and the training task may be or include a reconstruction task.

An autoencoder is a type of artificial neural network used to learn efficient embeddings of unlabelled input data. It is designed to compress the input data into a lower-dimensional representation and then reconstruct the original input data as closely as possible from this compressed representation. The process of learning to compress and reconstruct the data forces the autoencoder to capture the most salient features of the data in the embeddings, making it a powerful tool for feature learning. When entering input data, parts of the input data can be masked so that the autoencoder learns to reconstruct (unmask) the masked parts as well. The autoencoder thus learns to derive the missing parts from other parts of the input data. Other/further augmentation techniques are also possible (for example, sequence mutations, sequence shuffling, reverse complementing, windowing, cropping, noise injection, k-mer augmentation, homologous sequence, and/or others).

In addition to the reconstruction task, a machine learning model comprising an autoencoder can also solve one or more further training tasks. For example, the machine learning model can also be trained to perform classification and/or predict properties based on the embedding (see, e.g.: R. Xie et al.: A deep auto-encoder model for gene expression prediction, BMC Genomics 2017, 18(Suppl. 9): 845).

The machine learning model used to generate embeddings can be or include a transformer model. A transformer is a type of deep learning model that uses a mechanism called "attention" to dynamically weigh the relevance of different parts of an input sequence, enabling it to handle sequences of data with high efficiency and flexibility. Transformer models are used extensively in the field of natural language processing. Genome sequences can be interpreted as the language of biology, and therefore tools that can handle language data may be able to decipher the hidden patterns in these sequences.

The attention mechanism has revolutionized the way deep learning models process and interpret data. This technique was developed to circumvent the limitations of traditional recurrent models by providing a mechanism that attends to different parts of the input sequence as it generates the output. In the context of genomic data, this means that different genomic regions and their relationships can be considered dynamically during the interpretation process. The attention mechanism computes a weighted sum of input features, where the weightings, also referred to as attention values, are determined dynamically based on the input data. This mechanism allows the model to focus more on essential or relevant features and less on irrelevant or less important features (see, e.g.: S. R. Choi, M. Lee: Transformer Architecture and Attention Mechanisms in Genome Data Analysis: A Comprehensive Review, Biology 2023, 12, 1033).

The transformer model may be an autoregressive transformer. An autoregressive transformer refers to a type of transformer model that generates sequences in an autoregressive manner, meaning it predicts each element of the sequence one after another, using the previously generated elements as context.

Training a machine learning model may include the following steps:
for each gene dataset of a plurality of gene datasets:
- inputting the gene data into the machine learning model,
- obtaining output data from the machine learning model,
- quantifying a deviation between the output data and target data,
- reducing the deviation by modifying model parameters.

A loss function can be used to quantify the deviation. Different loss functions are known for different training tasks. The training can be terminated if one or more stop criteria are met. Such a stop criterion can be for example: a predefined maximum number of training steps/cycles/epochs has been performed, deviations between output data and target data can no longer be reduced by modifying model parameters, a predefined minimum of the loss function is reached, and/or an extreme value (e.g., maximum or minimum) of another performance value is reached.

When training a machine learning model, the dataset is usually divided into several subsets to ensure robust evaluation and generalization. The most common sets include a training set, a validation set and a test set. The training set is used to train the machine learning model. The validation set may be used to tune hyperparameters and make decisions about the model architecture. The validation set may help in evaluating the model's performance during training. The validation set allows for monitoring overfitting and making adjustments to improve model performance. The test set is usually kept separate from the training and validation sets and is used to assess the final performance of the model after training is complete. It provides an unbiased evaluation of how well the model is expected to perform on unseen data. There are various splitting strategies for dividing up the data, e.g., random split, k-fold cross-validation, and stratified split.

Once the model has been trained, it can be used to generate embeddings for new gene data. In this context, the term "new" means that the gene data usually (but not necessarily) was not used during training.

The machine learning model can be pre-trained. "Pre-training" is a phase in the machine learning process where a model is initially trained on a large dataset to learn general features and representations before being fine-tuned on a smaller, task-specific dataset. The pre-training task may be an auxiliary task on which the model can learn, e.g., the representation of the input data usually in an unsupervised or self-supervised learning. "Self-supervised learning" is a type of machine learning paradigm where the model is trained on a task that generates its own supervisory signal from the input data, without the need for explicit labelled data.

For example, the model can be pre-trained using training data from genes of different classes, orders, families, genera, and/or species (in the sense of biological taxonomy) of an organism and fine-tuned using training data from a specific class, order, family, genus, and/or species.

In an embodiment of the present disclosure, a gene embedding is generated using the Evo model, the Evo 2 model, or any similar model (see, e.g., E. Nguyen et al.: Sequence modeling and design from molecular to genome scale with Evo, Doi: 10.1101/2024.02.27.582234; G. Brixi et al.: Genome modeling and design across all domains of life with Evo 2, DOI: 10.1101/2025.02.18.638918). The Evo model is a genomic foundation model designed for sequence modeling and design across molecular to genome scales. It is based on advances in deep signal processing and is scaled to 7 billion parameters, supporting a context length of 131 kilobases at single-nucleotide resolution. Trained on whole prokaryotic genomes, Evo can generalize across fundamental modalities of molecular biology to perform zero-shot function prediction and multi-element generation tasks. The Evo model may be (re-)trained and/or finetuned on other gene data. The Evo 2 model is an advanced generative machine learning model with 7 billion and 40 billion parameters, trained on a vast dataset of genomic sequences. It excels in predicting mutational effects on protein function, non-coding RNA function, and overall organismal fitness.

In another embodiment of the present disclosure, the so-called Evo score and/or a similar score serves as a gene representation. The "Evo score" disclosed in E. Nguyen et al.: Sequence modeling and design from molecular to genome scale with Evo, Doi: 10.1101/2024.02.27.582234) refers to a metric for predicting gene essentiality based on changes in the Evo model's likelihood predictions. Specifically, the Evo score is computed as the difference in log-likelihoods between a mutated sequence and the unmutated wildtype sequence of a gene. This score is used to assess the impact of introducing mutations (e.g., premature stop codons) into the genome on the overall fitness or essentiality of genes within an organism. In the experiments described, mutations are introduced at the beginning of each coding sequence in a genome, and the Evo model predicts how these mutations affect the organism by comparing the likelihood of the mutated sequence to that of the original, unmutated sequence. The underlying hypothesis is that mutations to essential genes would result in larger, more negative changes in log-likelihood (Evo score), reflecting a higher impact on the organism's viability. This approach allows for a zero-shot prediction of gene essentiality across different genomes, leveraging the model's understanding of genomic context and sequence information without requiring explicit gene essentiality annotations for training. This perturbation emulates gene knock-out due to premature stop of the transcription, and it is related to the CRISPR knockout assay.

Instead of or in addition to the Evo score, an embedding of the region of interest may also be generated using the model. This embedding may be used as a gene representation.

As described above, the gene representation can also include a Gene Ontology (GO) annotation. However, for many genes there is no such GO annotation (yet). In an embodiment of the present disclosure, the gene representation is or includes a predicted GO annotation. The prior art discloses methods for predicting GO annotations (see, e.g.: P. Pinoli et al.: Computational algorithms to predict Gene Ontology annotations, BMC Bioinformatics, 2015, 16(Suppl. 6), S4; Ö Erten et al.: Predicting missing annotations in Gene Ontology with Knowledge Graph Embeddings and True Path Rule, SWAT4HCLS 2023: The 14th International Conference on Semantic Web Applications and Tools for Health Care and Life Sciences, February 13-16, 2023, Basel, Switzerland). Information about the protein encoded by the gene can also be incorporated into such a prediction (see, e.g.: G. A. Merino et al.: Hierarchical deep learning for predicting GO annotations by integrating protein knowledge, Bioinformatics, 38(19), 2022, 4488-4496).

In an embodiment of the present disclosure, the GO annotation is predicted based on the amino acid sequence of the protein encoded by the gene. Such a GO annotation prediction can also be considered a protein representation because it is based on information about the protein. However, for all representations described in this disclosure that relate to a gene or a gene product (e.g. the protein encoded by the gen), the reference to the gene or gene product should not be understood as a limitation to the effect that the representation is based solely on information about the gene or gene product.

In an embodiment of the present disclosure, multiple (i.e., more than one) gene representations are generated.

Another numerical representation that is generated is a product representation. The product representation is a numerical representation of the gene product. In an embodiment of the present disclosure, the gene product is the protein encoded by the gene and the product representation is a protein representation. The protein representation is a numerical representation of the protein encoded by the gene.

The protein representation may represent the structure of the protein and/or may be derived from the structure of the protein. The structure of a protein can be the primary structure, the secondary structure, and/or the tertiary structure.

The primary structure refers to the sequence of amino acids that make up the protein.

The secondary structure of a protein refers to the regular, recurring patterns of folding and arrangement of the protein's polypeptide chain. These patterns primarily involve interactions between nearby amino acids within the linear sequence. The two most common types of secondary structures in proteins are alpha helices and beta sheets.

The tertiary structure of a protein refers to the three-dimensional arrangement and folding of the entire polypeptide chain, including all its secondary structures (such as alpha helices and beta sheets), into a unique and specific shape. This intricate, folded structure is often critical for the protein's overall function and may determine its active site, binding sites, and/or interaction with other molecules.

In an embodiment of the present disclosure, the protein representation may comprise and/or be derived from the sequence of amino acids in the protein.

For example, the sequence of amino acids in an amino acid sequence (protein sequence) can be represented by one-hot encoding.

One-hot encoding treats all amino acids equally. There are other methods of representation that incorporate knowledge of amino acids and/or amino acid sequences.

One example is the BLOcks SUbstitution Matrix (BLOSUM) representing each amino acid by its corresponding row in the BLOSUM matrix (see, e.g., S. Henikoff, J.G. Henikoff: Amino acid substitution matrices from protein blocks, Proc Natl Acad Sci USA, 1992, 15; 89). The BLOSUM matrix derived from protein sequence alignments keeps the evolutionary information about which pairs of amino acids are easily interchangeable during evolution.

Another example is the principal components score Vector of Hydrophobic, Steric, and Electronic properties (VHSE8) which captures physicochemical properties (see, e.g., H. Mei et al.: A new set of amino acid descriptors and its application in peptide QSARs, Peptide Science, 80 (2005) 6, 775-786).

Other examples of protein representations based on the protein's amino acid sequence are described in the literature (see, e.g.: ElAbd et al.: Amino acid encoding for deep learning Applications, BMC Bioinformatics, 2020, 21:235; W. Wilman et al.: Machine-designed biotherapeutics: opportunities, feasibility and advantages of deep learning in computational antibody discovery, Briefings in Bioinformatics, 2022, 23(4), 1-20).

The representation of a protein can also be generated using a (pre-)trained machine learning model. Such models can be (pre-)trained, e.g., in a self-supervised learning procedure based on a variety of naturally occurring amino acid sequences to reconstruct amino acid sequences in which a portion of the amino acids have been masked. In addition to or instead of masking, other augmentation techniques can also be used (see, for example, the augmentation techniques mentioned above). The embedding from which the machine learning model reconstructs the amino acid sequence can be used as a representation of the protein.

Such a (pre-)trained machine learning model can be a protein language model, also referred to as Large Language Model (LLM) (see, e.g.: D. Ofer et al.: The language of proteins: NLP, machine learning & protein sequences, Computational and Structural Biotechnology Journal, 19, 2021, 1750-1758). Protein language models are machine learning models based on natural language processing methods, especially attention and transformers. They can be (pre-)trained on large ensembles of protein sequences, and usually capture long-range dependencies within a protein sequence. These (pre-)trained models are able to predict protein sequences in an unsupervised way, either taking as input a single sequence or a multiple sequence alignment (MSA), potentially by transferring knowledge from their (large) training set. Examples of such models are: ESM-1b (A. Rives et al.: Biological structure and function emerge from scaling unsupervised learning to 250 million protein sequences, Proceedings of the National Academy of Sciences, 118(15), 2021), ESM-2 (Z. Lin et al.: Evolutionary-scale prediction of atomic-level protein structure with a language model, Science, 2023, Vol. 379, Issue 6637, pp. 1123-1130), ProtT5-XL-BFD (see, e.g.; A. Elnaggar et al.: ProtTrans: Towards Cracking the Language of Life's Code Through Self-Supervised Deep Learning and High Performance Computing, arXiv:2007.06225).

Such a pre-trained machine learning model for generating a protein embedding can also be or comprise a convolutional neural network (CNN) (see, e.g., Y. LeCun et al.: Deep learning, Nature 521, 436 (2015); K.K. Yang et al.: Convolutions are competitive with transformers for protein sequence pretraining, DOI: 10.1101/2022.05.19.492714).

Such a pre-trained machine learning model may be or comprise a long short-term memory (LSTM) (see, e.g., I. Sutskever et al.: Sequence to sequence learning with neural networks, Adv. Neural Inf. Process. Syst., 2014, 2, 3104-3112).

Such a pre-trained machine learning model for generating a protein embedding can also be a model that combines different representation learning techniques (see, e.g., V. Gligorijević *et al*.: *Structure-based protein function prediction using graph convolutional networks,* Nat Commun 12, 3168 (2021)).

The pre-trained machine learning model may have been trained to predict protein structure based on amino acid sequence. Such a structural model generates embeddings of proteins in which information about the three-dimensional arrangement of atoms in space is encoded.

This three-dimensional structure usually is crucial for the protein to carry out its biological functions. The primary structure of a protein is determined by the sequence of amino acids in the protein chain. However, the final, functional structure of a protein is not a linear chain but a complex three-dimensional shape. This folded structure is dictated by the interactions between the amino acid residues, including covalent bonds and non-covalent forces such as hydrogen bonds, ionic interactions, and hydrophobic interactions.

Models that generate protein representations in which structural information of proteins is encoded are described, for example, in: M. Heinzinger et al.: ProstT5: Bilingual Language Model for Protein Sequence and Structure, DOI: 10.1101/2023.07.23.550085; T. Hayes et al.: Simulating 500 million years of evolution with a language model, DOI: 10.1101/2024.07.01.600583).

Many of the available pre-trained machine learning models have been trained based on human and/or animal proteins. Such a pre-trained machine learning model may be re-trained (finetuned) on proteins of the organism under investigation and/or similar organisms (e.g., organisms of the same class, order, family, and/or genus). For example, it may make a difference whether a model was trained on the basis of training data from eukaryotes or on the basis of training data from prokaryotes. Prokaryotes are single-celled organisms that lack a nucleus and other membrane-bound organelles. The protein sequences in prokaryotes, such as bacteria, tend to be shorter and less complex. Prokaryotic proteins often have simpler structures and functions. Eukaryotes are organisms whose cells contain a nucleus and other membrane-bound organelles. Their genetic material is enclosed within a nuclear membrane. Eukaryotic proteins are generally longer and more complex, with diverse post-translational modifications and intricate folding patterns. Therefore, it may be useful to finetune a pre-trained model based on training data that is more closely adapted to the organism under consideration.

In an embodiment of the present disclosure, multiple (i.e., more than one) protein representations are generated.

If the gene product is a small peptide, the methods described herein for generating a protein representation can also be applied to generate a peptide representation.

If the gene product is an RNA molecule, the methods described herein for generating a gene representation can also be applied to generate an RNA representation.

In an embodiment of the present disclosure, multiple (i.e., more than one) product representations are generated.

Different product representations may represent different gene products or represent the same gene product in different ways.

Another numerical representation that is generated is an interrelation representation. The interrelation representation is a numerical representation of interrelations of the gene and/or gene product with other genes and/or gene products of the organism.

In organisms, genes and gene products (e.g. proteins) do not function independently; there are numerous interactions between genes/gene products that maintain the stability of the internal environment. The complex biological networks consisting of interacting genes/gene products are generally scale-free, meaning there are few highly connected nodes and many rarely connected nodes in the networks.

In scale-free networks, removing highly connected nodes is more likely to disrupt network connectivity or increase the shortest path length between nodes. Highly connected nodes are more important for maintaining the functions of scale-free biological networks or the stability of organisms than rarely connected nodes. This means that the essentiality of genes/gene products can be predicted by analysing the topological properties of nodes in biological networks.

In an embodiment of the present disclosure, the interrelation representation is or comprises a protein-protein interaction network and/or interrelation data obtained therefrom.

A protein-protein interaction network is a graphical representation that illustrates the interactions between proteins within a cell or a specific biological context. These interactions are crucial for understanding cellular processes, as proteins often function by interacting with other proteins to carry out biological activities such as signal transduction, cellular transport, and immune responses. In a protein-protein interaction network, nodes usually represent proteins, and edges usually represent the interactions between them. Edges can be undirected, indicating a bidirectional interaction, or directed if the directionality of the interaction is known. Nodes and edges can be annotated with additional information, such as the type of interaction, experimental method used to detect the interaction, biological function, and/or cellular location.

Data on protein-protein interactions can be gathered from various sources. Biochemical assays such as yeast two-hybrid screening, co-immunoprecipitation, and fluorescence resonance energy transfer (FRET) directly test the physical interactions between proteins. There are several curated databases that compile experimentally verified protein-protein interactions from the literature. Examples include the Biological General Repository for Interaction Datasets (BioGRID), the Human Protein Reference Database (HPRD), and the Database of Interacting Proteins (DIP). Computational methods can predict protein-protein interactions based on various criteria, including gene co-expression, genetic interactions, domain-domain interactions, and machine learning models trained on known protein-protein interactions.

Removing proteins often brings huge changes into a network, such as causing protein-protein interaction networks to rapidly collapse into isolated nodes or clusters, which may break the function of the modules and/or the interactions in key biological processes. Therefore, the essentiality of protein is closely related to its topological characteristics in protein-protein interaction networks. Topology-based methods score proteins by their centralities in a protein-protein interaction network.

There are numerous score/values that can be derived from a protein-protein interaction network and that provide information about the essentiality of a protein (see, e.g., X. Li et al.: Network-based methods for predicting essential genes or proteins: a survey, Briefings in Bioinformatics, 21(2), 2020, 566-583); all of these scores/values can be used in the context of the present disclosure.

Examples of scores/values that can be derived from a network (in general, not only from a protein-protein interaction network) are: degree of a node (i.e., number of connections (edges) it has to other nodes), betweenness centrality (i.e., the extent to which a node lies on paths between other nodes), closeness centrality (i.e., how close a node is to all other nodes in the network), clustering coefficient (i.e., degree to which nodes in a network tend to cluster together), modularity (i.e., strength of division of a network into modules), path length (i.e., the average shortest path length in a network), Eigenvector centrality (i.e., a measure that considers not only the number of connections a node has but also the importance of the nodes it is connected to), edge density (i.e., ratio of the number of actual edges in the network to the number of possible edges), feedback loops (i.e., the presence and nature of feedback loops (positive or negative)).

In another embodiment of the present disclosure, the interrelation representation is a representation of interrelations of the gene with other genes of the organism.

Similar to protein-protein interaction networks, interrelation networks can also be generated for genes. Such networks can indicate relationships between genes. For example, such a network can be a gene co-expression network or include it.

A gene co-expression network is a representation that maps the relationships between genes based on their expression patterns across various conditions, tissues, and/or developmental stages. In this network, nodes represent genes, and edges between nodes indicate a correlation in their expression levels, suggesting that these genes may be co-regulated and/or involved in related biological processes.

So, the interrelation representation may be or comprise a gene co-expression network and/or interrelation data obtained therefrom.

The connections (edges) in a gene co-expression network are typically established based on statistical measures of correlation (such as Pearson or Spearman correlation coefficients) between the expression levels of gene pairs. A high correlation coefficient between two genes suggests that their expression levels rise and fall together across the conditions studied.

These networks are usually undirected because the correlation measures used to construct them do not imply causation or directionality in the relationship between genes.

Gene co-expression networks often contain clusters or modules of genes that are more tightly connected to each other than to genes outside the cluster. These modules can indicate groups of genes that work together in specific biological functions or pathways.

For generating a gene co-expression network, gene expression data may be collected across various samples, which may be different tissue types, developmental stages, and/or treatment conditions. This data may be obtained from high-throughput techniques like microarrays or RNA sequencing (RNA-seq). The expression data is usually normalized and processed to ensure comparability across samples. This may involve adjusting for batch effects, filtering out lowly expressed genes, and transforming the data to reduce skewness. Pairwise correlation coefficients between all possible gene pairs may be calculated to quantify the similarity in their expression patterns across the samples. A network may then be constructed where each node represents a gene. Edges may be added between gene pairs that may have correlation coefficients above a certain threshold, indicating significant co-expression.

Genes that serve as hubs, meaning they have a high degree of connectivity with many other genes, can be considered essential. These hub genes often play crucial regulatory roles in maintaining cellular functions and homeostasis.

Gene co-expression networks often contain modules or clusters of genes that are highly interconnected and co-expressed. These modules can correspond to biological pathways or processes. Identifying genes that are central within modules critical for cell survival, growth, or reproduction can highlight essential genes that are key to these processes.

By comparing gene co-expression networks across different species, particularly those that are conserved across evolutionary distances, one can identify genes that are consistently positioned as hubs or within important modules. Such genes are likely to be essential for fundamental biological functions.

Interrelation data derived from a gene co-expression network is, for example, a node centrality measure, module membership, and/or other/further topological properties (see, e.g., B. Zhang, S. Horvath: A General Framework for Weighted Gene Co-expression Network Analysis, Statistical Applications in Genetics and Molecular Biology, 2005).

In an embodiment of the present disclosure, a number of nearest neighbours is identified for a gene of interest (e.g., 2, 3, 4, 5, 6, 7, 8, 9, 10, or more than 10 nearest neighbours). The correlation coefficients between the gene of interest and its closest neighbours can be summarized in a vector (e.g. in ascending or descending order). If a gene under consideration has fewer than the selected number of nearest neighbours, the corresponding values of the vector representing correlation coefficients can be set to zero. If a gene under consideration has more than the selected number of nearest neighbours, the number of edges connecting the gene to other genes can be included in the vector. An expression level of the gene of interest can be included in such a vector (e.g. in the form of a mean TPM value (TPM: Transcripts Per Million). In this way, a feature vector can be generated that always has the same dimension for any genes. However, there are numerous other ways to combine information from a gene co-expression network into a numerical representation of a fixed size for the gene.

In another embodiment of the present disclosure the interrelation representation is or comprises a gene regulatory network or interrelation data obtained therefrom.

A gene regulatory network describes regulatory relationships between genes, gene products, and/or other molecules within a cell. A gene regulatory network can be represented as a graph where nodes represent genes, gene products, or other molecules, and edges represent regulatory interactions between them (activation or inhibition). Genes that serve as hubs with a high degree of connectivity are often crucial for maintaining the network's integrity and functionality. Identifying these hub genes can highlight candidates for essential genes, as their disruption could lead to cascading effects throughout the network, potentially compromising cell viability and/or function. Gene regulatory networks often exhibit modular structures, where a module consists of a group of genes that are highly interconnected and co-regulated. Modules may correspond to specific biological processes or pathways. Genes that play central roles within critical modules, especially those involved in fundamental cellular processes such as DNA replication, transcription, translation, and/or cell cycle control, are likely to be essential.

There are numerous score/values that can be derived from a gene regulatory network and that provide information about the essentiality of a gene (see, e.g., V. A. Huynh-Thu, G. Sanguinetti: Gene Regulatory Network Inference: An Introductory Survey, Methods in Molecular Biology, 2019, Vol. 1883, Humana Press, New York); all of these scores/values can be used in the context of the present disclosure.

Another numerical representation that is generated is a gene expression response representation. The gene expression response representation is a numerical representation of one or more responses of an expression level of the gene to one or more external stimuli.

External stimuli can be, for example, environmental stressors (e.g., temperature changes, oxidative stress, osmotic stress, pH change) nutritional changes (e.g., glucose or carbon source availability, starvation conditions, presence or absence of specific vitamins or minerals), chemical compounds (e.g., crop protections products, toxins, poisons, hormones, growth factors, heavy metals), biological factors (e.g., pathogen infection, interaction with symbiotic partners, exposure to pheromones or other signalling molecules from cospecies or other species), mechanical stimuli (e.g., shear stress, stretch or compression, vibration) light (e.g., light intensity, light wavelength (in particular ultraviolet light, exposure time), and/or ionizing radiation.

In an embodiment of the present disclosure, the gene expression response representation is a representation of the expression response of the gene to treatment with one or more chemical compounds.

Such a chemical compound may be or comprise an active ingredient of a crop protection product that is commonly used to control the organism and/or a similar organism. Gene expression responses can be determined for several (2, 3, 4, 5, 6, 7, 8, 9, 10 or more than ten) such active ingredients, whereby the active ingredients may have different modes of action.

However, it may also be one or more other chemical compounds, e.g. one or more chemical compounds that are known to have an effect on gene expression in the organism or a similar organism.

The response of gene expression to an external stimulus can be determined experimentally in a variety of ways, for example by RNA sequencing.

RNA sequencing (RNA-seq) is a high-throughput sequencing technology used to analyze the complete set of RNA transcripts produced by the genome of an organism. In the context of determining the response of gene expression to external stimuli, such as treatment with a chemical compound, RNA-seq enables researchers to quantitatively assess changes in the transcriptome. By comparing RNA profiles from treated and untreated samples, RNA-seq facilitates the identification of differentially expressed genes (DEGs) that are upregulated or downregulated in response to the external stimulus.

Single-Cell RNA Sequencing (scRNA-seq) analyzes the expression levels of genes in individual cells, providing a high-resolution view of cellular responses to external stimuli. This technique can uncover heterogeneity in responses among cells in a population and identify specific cell types or states that are particularly responsive to a stimulus.

Quantitative Real-Time PCR (qRT-PCR) is a sensitive and precise method for quantifying gene expression levels. It is often used to validate findings from microarray or RNA-seq experiments. By designing specific primers for target genes, researchers can measure the fold change in gene expression in response to stimuli with high accuracy.

The experimentally determined data can be converted into a numerical representation in different ways.

The experimental data may include, for example, gene expression levels as a function of time and external stimulus. A gene expression level can be expressed as transcripts per million (TPM). "Transcripts per million" refers to a normalization method used, e.g., in RNA sequencing data analysis to quantify gene expression levels. TPM accounts for both the sequencing depth (the total number of reads) and the length of the transcripts, allowing for a more accurate comparison of gene expression levels across different samples.

For example, the gene expression level can be expressed as a mathematical function of the amount of chemical compound and time. The mathematical function may be a linear or non-linear function. The parameters of the mathematical function can be determined by regression, for example. The parameters of the mathematical function can be summarized in a feature vector. The feature vector is a numerical representation of the response of gene expression level to treatment with a chemical compound.

Other methods are also possible (see, for example Y. Poeschl et al.: Explaining gene responses by linear modeling, German conference on bioinformatics 2014, ISBN: 978-3-88579-629-9, pp 27-35; Y. F. Brun et al.: Simultaneous modeling of concentration-effect and time-course patterns in gene expression data from microarrays, Cancer Genomics & Proteomics, 2008, 5(1): 43-53).

Besides the mentioned representations, further representations can be generated. An example of such a further representation is an organism representation that represents the organism. Another example is a cell representation that represents the cell in which the gene is expressed and/or the gene product is produced.

In a further step, the individual representations (on ore more gene representation(s), product representation(s), interrelation representation(s), gene expression response representation(s)) and optionally one or more further representation(s) are merged (combined) into a joint candidate representation.

Merging different representations into a single, unified representation is a common task in machine learning. The goal is to combine information from multiple sources and/or feature sets into a comprehensive representation that captures the essence of the data more effectively than any single representation could. Example techniques for merging representations are concatenation, averaging, weighted averaging, principal component analysis (PCA), autoencoder, attentions mechanism, and/or neural network fusion.

In a further step, a prediction is carried out: based on the candidate representation, it is predicted how essential the candidate is for the organism.

The prediction may be a classification: based on the candidate representation, the candidate is assigned to one of at least two classes.

One of the at least two classes may be a class representing genes/gene products that are essential. Another one of the at least two classes may be a class representing genes/gene products that are not essential.

In an embodiment of the present disclosure, there are exactly two classes (binary classification): one class representing essential genes/gene products, and the other class representing non-essential genes/ gene products.

In another embodiment, there are more than two classes (multi-class classification). Different classes may represent different degrees of essentiality. Different classes may also represent different levels of probability that a gene/gene product is essential.

The prediction of essentiality can be a regression: based on the candidate representation, an essentiality score can be predicted for the candidate. For example, the essentiality score can correlate positively with essentiality: the higher the essentiality score, the greater the essentiality.

The essentiality score can indicate the probability that the candidate is essential.

The prediction is carried out with the help of a trained machine learning model.

The machine learning model was trained based on training data. The training data comprised, for each reference of a plurality of references, (i) a gene representation, a product representation, an interrelation representation, a gene expression response representation and optionally one or more further representations, and (ii) an information about the essentiality of the reference.

The term "plurality" means more than 10, e.g. more than 100 or even more than 1000.

A "reference" relates to a gene and corresponding gene product, of which data was used to train the machine learning model. While it is known for the "reference" whether the gene and/or the gene product are essential and/or how essential the gene and/or the gene product are, it is not known for the candidate.

The gene of the reference can also be referred to as the "reference gene". The gene product of the reference can also be referred to as the "reference product gene". Such a nomenclature can be helpful in preventing clarity objections in examination proceedings of the present patent application. If this nomenclature is continued, the gene representation of the reference gene can also be referred to as the "reference gene representation", the product representation of the reference gene product can also be referred to as the "reference product representation", the gene expression response representation of the reference gene can also be referred to as the "reference gene expression response representation", and the interrelation representation of the reference gene and/or reference gene product can be also referred to as the "reference interrelation representation".

However, the readability of the following explanations would be impeded by retaining this nomenclature, which is why the nomenclature was not consistently applied. However, the context makes it clear which representations are meant.

It can therefore be stated that the same data is collected for the reference as for the candidate. Everything that has been written about the candidate's representations can be applied to the reference's representations.

The reference representations are merged into a joint reference representation, as described for the candidate.

The joint reference representation serves as input data and the information on the essentiality of the reference serves as target data (ground truth) during training.

It is also possible for the different representations of a refence to serve as input data (gene representation, product representation, interrelation representation, gene exposure response representation, one or more further representations). It is possible that the machine learning model is trained to merge the different representations into a joint representation.

Training of the machine learning model typically involves the following steps:
For each reference of the plurality of references:
- inputting the input data into the machine learning model, wherein the machine learning model is configured to determine predicted information about the essentiality of the reference based on the input data and on model parameters,
- receiving the predicted information,
- quantifying a deviation between the predicted information and the target data,
- reducing the deviation by modifying the model parameters,
- storing the trained machine learning model and/or the model parameters, and/or outputting the trained machine learning model and/or the model parameters, and/or transmitting the trained machine learning model and/or the model parameters to a separate computer system, and/or using the trained machine learning model to predict essentiality of a candidate.

A loss function can be used to quantify the deviation. Different loss functions are known for different training tasks. If the model is a binary classification model, Binary Cross Entropy Loss may be used as loss function, for example. If the model is a multi-class classification model, Cross-Entropy Loss may be used as the loss function, for example. If the model is a regression model, Mean Squared Error (MSE) may be used as loss function, for example.

The loss function is usually minimized using an optimization method, e.g. a gradient descent method.

The training can be terminated if one or more stop criteria are met. Such a stop criterion can be for example: a predefined maximum number of training steps/cycles/epochs has been performed, deviations between output data and target data can no longer be reduced by modifying model parameters, a predefined minimum of the loss function is reached, and/or an extreme value (e.g., maximum or minimum) of another performance value is reached.

Once the machine learning model has been trained, it can be used to predict essentiality for the candidate.

The candidate representation is fed into the trained machine learning model as input data.

The trained machine learning model predicts how essential the candidate's gene and/or gene product is to the organism and outputs that information.

This information may be a predicted class. This information may be a predicted essentiality score. This information may be a predicted probability that the candidate's gene and/or gene product is essential.

This information can be output, i.e. displayed on a monitor and/or printed out with a printer and/or stored in a data memory and/or transmitted to a separate computer system.

If the information is an essentiality score or an essentiality probability, such a value can be compared to a threshold. The threshold can define a minimum essentiality that must be reached for the candidate to be pursued. Such a threshold can be set by a user and/or pre-defined by an expert. If the value does not reach the minimum essentiality, the candidate can be discarded. If the value reaches or exceeds the minimum essentiality, the candidate is a possible target for a crop protection product.

Once a target is hypothesized, in vitro experiments may be conducted to study the target's function. This may involve expressing the target protein in a model system and using small molecules or RNA interference (RNAi) to inhibit its function. Observing the effects of these interventions helps in understanding the target's role. In a further step, in vivo studies may be performed, where the target's role is validated in the actual pest and/or a model organism. This could involve genetic modification techniques like CRISPR/Cas9 to knock out the target gene and observe the effects on the pest's development, reproduction, and/or survival. Alternatively, specific inhibitors can be applied to the pest to observe the phenotypic effects of target modulation. It's crucial to validate the target not just in the pest but also in the context of the crop. This involves testing the target modulation strategy (e.g., using a specific inhibitor and/or genetic modification) in a controlled environment where the pest infests the crop. The aim is to observe whether the crop protection strategy effectively protects the crop without adverse effects on the crop itself or the environment. Even after a target is validated, its selectivity and safety must be thoroughly assessed. This involves ensuring that the target or the strategy used to modulate it does not harm non-target organisms, including beneficial insects, animals, and humans. Regulatory and environmental safety assessments are critical at this stage. With a validated target, the next steps may involve optimizing the intervention strategy (e.g., chemical formulation, delivery system) and conducting extensive field trials to ensure efficacy, safety, and practicality under real-world conditions.

The subject matter of the present disclosure will be explained in more detail below with the aid of drawings, without intending to limit the subject matter to the features and combinations of features depicted in the drawings.

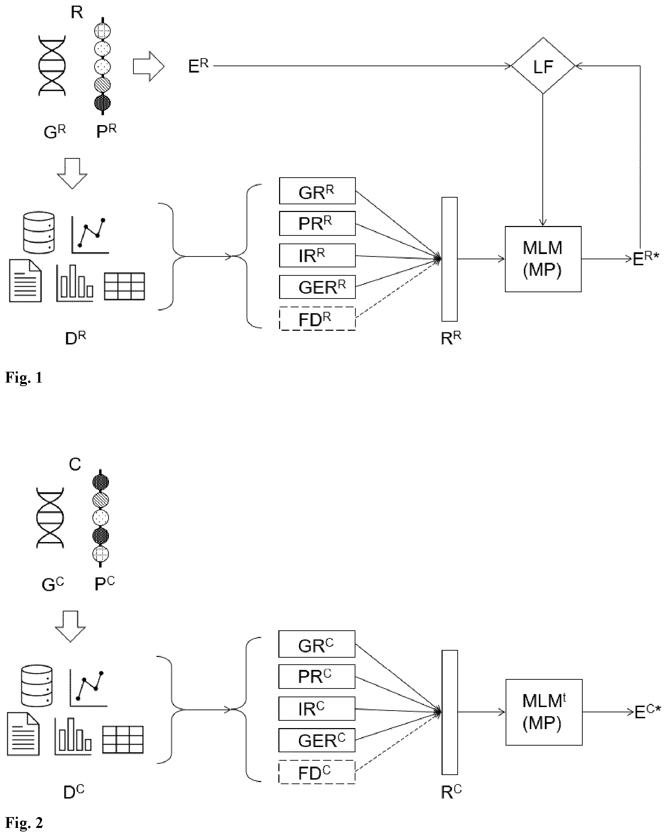

Fig. 1 shows an example of how to train the machine learning model of the present disclosure.

Training is done with training data. The training data represents a plurality of references. In Fig. 1, one such reference R is shown. The reference R is characterized by a gene G^{R} and a gene product P^{R}. The gene product P^{R} may be the protein that is encoded by the gene G^{R}. The reference R relates to an organism. The organism may be a plant pest.

For the reference R, it is known whether and/or how essential the gene G^{R} and/or gene product P^{R} is for the organism. This essentiality E^{R} is used as target data when training the machine learning model MLM.

A variety of data D^{R} is collected for the reference R. Based on the data D^{R}, a gene representation GR^{R}, a product representation PR^{R}, an interrelation representation IR^{R} and a gene expression response representation GER^{R} are generated.

The gene representation GR^{R} is a numerical representation of the gene G^{R}. The product representation PR^{R} is a numerical representation of the gene product P^{R}. The interrelation representation IR^{R} is a numerical representation of interrelations of the gene G^{R} and/or gene product P^{R} with other genes and/or gene products of the organism. The gene expression response representation GER^{R} is a numerical representation of one or more responses of an expression level of the gene G^{R} to one or more external stimuli.

Optionally, further data is provided in a further numerical representation FD^{R}. The further representation FD^{R} can, for example, represent the organism and/or its properties.

The gene representation GR^{R}, the product representation PR^{R}, the interrelation representation IR^{R}, the gene expression response representation GER^{R}, and, if present, the further representation FD^{R} are merged into a joint reference representation R^{R}.

The reference representation R^{R} is fed to the machine learning model MLM as input data. It is also possible to feed one or more of the mentioned representations separately to the machine learning model MLM and to train the machine learning model MLM to merge the separately fed representations.

The machine learning model MLM is configured to predict the essentiality of the reference R based on the reference representation R^{R} and model parameters MP. The machine learning model MLM outputs a predicted essentiality E^{R}*. A loss function LF is used to quantify a deviation between the known essentiality E^{R} and the predicted essentiality E^{R}*.

The deviation can be reduced by modifying model parameters, e.g. in an optimization procedure.

The steps mentioned are carried out for a plurality of references. In this way, the machine learning model MLM learns to predict the essentiality of a gene and/or a protein encoded by the gene. The result of the training is a trained machine learning model that can be used for prediction.

Fig. 2 shows an example of the use of a trained machine learning model of the present disclosure for prediction.

The trained machine learning model MLM^{t} may have been trained as described in relation to Fig. 1.

The starting point for the prediction is a candidate C. The candidate C is characterized by a gene G^{C} and a gene product P^{C}. The gene product P^{C} may be the protein that is encoded by the gene G^{C}. The candidate C relates to an organism. The organism is a plant pest.

A variety of data D^{C} is collected for the candidate C. Based on the data D^{C}, a gene representation GR^{C}, a product representation PR^{C}, an interrelation representation IR^{C} and a gene expression response representation GER^{C} are generated.

The gene representation GR^{C} is a numerical representation of the gene G^{C}. The product representation PR^{C} is a numerical representation of the gene product P^{C}. The interrelation representation IR^{C} is a numerical representation of interrelations of the gene G^{C} and/or gene product P^{C} with other genes and/or gene products of the organism. The gene expression response representation GER^{C} is a numerical representation of one or more responses of an expression level of the gene G^{C} to one or more external stimuli.

If further data was used as input data for training the machine learning model, further data may be provided in a further numerical representation FD^{C}. The further representation FD^{C} may, for example, represent the organism and/or its properties.

The gene representation GR^{C}, the product representation PR^{C}, the interrelation representation IR^{C}, the gene expression response representation GER^{C}, and, if present, the further representation FD^{C} are merged into a joint candidate representation R^{C}.

The candidate representation R^{C} is fed to the trained machine learning model MLM^{t} as input data.

If the machine learning model was trained to merge representations, these are fed separately into the trained machine learning model MLM^{t}.

The trained machine learning model MLM^{t} is configured (and was trained) to predict the essentiality of the candidate C based on the candidate representation R^{C} and model parameters MP. The trained machine learning model MLM^{t} outputs a predicted essentiality E^{C}*.

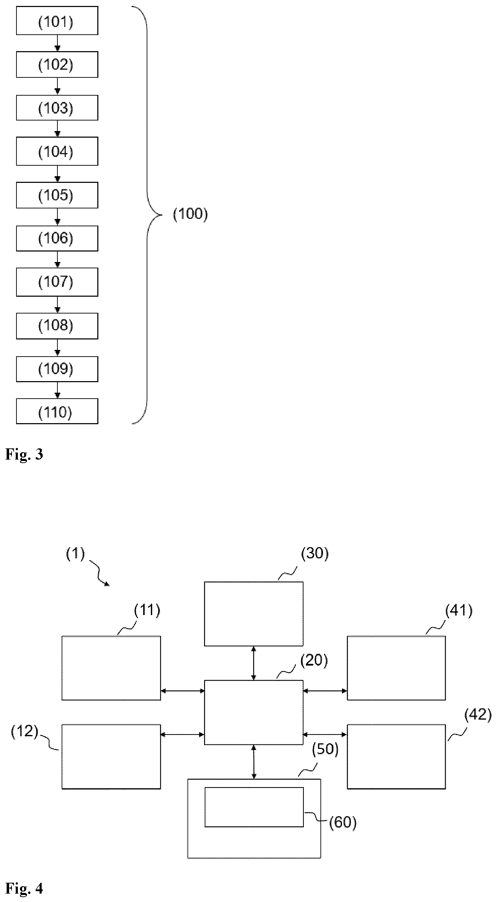

Fig. 3 shows an embodiment of the computer-implemented method in the form of a flowchart.

The method (100) comprises the following steps:

| | |
|---|---|
| (101) | determining a candidate, wherein the candidate is related to a gene and a gene product of a pest organism, |
| (102) | generating a gene representation, wherein the gene representation is a numerical representation of the gene, |
| (103) | generating a product representation, wherein the product representation is a numerical representation of the gene product, |
| (104) | generating an interrelation representation, wherein the interrelation representation is a numerical representation of interrelations of the gene and/or gene product with other genes and/or gene products of the organism, |
| (105) | generating a gene expression response representation, wherein the gene expression response representation is a numerical representation of one or more responses of an expression level of the gene to one or more external stimuli, |
| (106) | generating a candidate representation based on the gene representation, the product representation, the interrelation representation, and the gene expression response representation, |
| (107) | providing a trained machine learning model, wherein the machine learning model is configured and was trained to determine how essential a gene and/or gene product is for an organism, |
| (108) | inputting the candidate representation into the trained machine learning model, |
| (109) | receiving information about how essential the gene and/or gene product is to the organism as an output from the trained machine learning model, |
| (110) | outputting the information. |

The operations in accordance with the teachings herein may be performed by at least one computer system specially constructed for the desired purposes or general-purpose computer specially configured for the desired purpose by at least one computer program stored in a typically non-transitory computer readable storage medium.

A "computer system" is a system for electronic data processing that processes data by means of programmable calculation rules. Such a system usually comprises a "computer", that unit which comprises a processor for carrying out logical operations, and also peripherals.

In computer technology, "peripherals" refer to all devices which are connected to the computer and serve for the control of the computer and/or as input and output devices. Examples thereof are monitor (screen), printer, scanner, mouse, keyboard, drives, camera, microphone, loudspeaker, etc. Internal ports and expansion cards are, too, considered to be peripherals in computer technology.

Computer systems of today are frequently divided into desktop PCs, portable PCs, laptops, notebooks, netbooks and tablet PCs and so-called handhelds (e.g. smartphone); all these systems can be utilized for carrying out the computer-implemented method of the present disclosure.

The term "non-transitory" is used herein to exclude transitory, propagating signals or waves, but to otherwise include any volatile or non-volatile computer memory technology suitable to the application.

The term "computer system" should be broadly construed to cover any kind of electronic device with data processing capabilities, including, by way of non-limiting example, personal computers, servers, embedded cores, computing system, communication devices, processors (e.g., digital signal processor (DSP)), microcontrollers, field programmable gate array (FPGA), application specific integrated circuit (ASIC), etc.) and other electronic computing devices.

The term "process" as used above is intended to include any type of computation or manipulation or transformation of data represented as physical, e.g., electronic, phenomena which may occur or reside e.g., within registers and/or memories of at least one computer system or processor. The term processing unit includes a single processor or a plurality of distributed or remote such units.

Fig. 4 illustrates a computer system (1) according to some example implementations of the present disclosure in more detail. The computer system may include one or more of each of a number of components such as, for example, a processing unit (20) connected to a memory (50) (e.g., storage device).

The processing unit (20) may be composed of one or more processors alone or in combination with one or more memories. The processing unit (20) is generally any piece of computer hardware that is capable of processing information such as, for example, data, computer programs and/or other suitable electronic information. The processing unit (20) is composed of a collection of electronic circuits some of which may be packaged as an integrated circuit or multiple interconnected integrated circuits (an integrated circuit at times more commonly referred to as a "chip"). The processing unit (20) may be configured to execute computer programs (60), which may be stored onboard the processing unit or otherwise stored in the memory (50) of the same or another computer.

The processing unit (20) may be a number of processors, a multi-core processor or some other type of processor, depending on the particular implementation. Further, the processing unit (20) may be implemented using a number of heterogeneous processor systems in which a main processor is present with one or more secondary processors on a single chip. As another illustrative example, the processing unit (20) may be a symmetric multi-processor system containing multiple processors of the same type. In yet another example, the processing unit (20) may be embodied as or otherwise include one or more ASICs, FPGAs or the like. Thus, although the processing unit (20) may be capable of executing a computer program (60) to perform one or more functions, the processing unit (20) of various examples may be capable of performing one or more functions without the aid of a computer program (60). In either instance, the processing unit (20) may be appropriately programmed to perform functions or operations according to example implementations of the present disclosure.

The memory (50) is generally any piece of computer hardware that is capable of storing information such as, for example, data, images, computer programs (e.g., computer-readable program code (60)), machine learning models and/or other suitable information either on a temporary basis and/or a permanent basis. The memory may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another. Computer-readable medium as described herein may generally refer to a computer-readable storage medium or computer-readable transmission medium.

In addition to the memory (50), the processing unit (20) may also be connected to one or more interfaces for displaying, transmitting and/or receiving information. The interfaces may include one or more communications interfaces and/or one or more user interfaces. The communications interface(s) may be configured to transmit and/or receive information, such as to and/or from other computer(s), network(s), database(s), camera(s) or the like. The communications interface may be configured to transmit and/or receive information by physical (wired) and/or wireless communications links. The communications interface(s) may include interface(s) (41) to connect to a network, such as using technologies such as cellular telephone, Wi-Fi, satellite, cable, digital subscriber line (DSL), fiber optics and the like. In some examples, the communications interface(s) may include one or more short-range communications interfaces (42) configured to connect devices using short-range communications technologies such as NFC, RFID, Bluetooth, Bluetooth LE, ZigBee, infrared (e.g., IrDA) or the like.

The user interfaces may include a display (30). The display (30) may be configured to present or otherwise display information to a user, suitable examples of which include a liquid crystal display (LCD), light-emitting diode display (LED), plasma display panel (PDP) or the like. The user input interface(s) (11) may be wired or wireless, and may be configured to receive information from a user into the computer system (1), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology (12) for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers, cameras and the like.

As indicated above, a computer program (60) may be stored in memory (50), and executed by processing unit (20) that is thereby programmed, to implement functions of the systems, subsystems, tools and their respective elements described herein. As will be appreciated, any suitable program code instructions may be loaded onto a computer or other programmable apparatus from a computer-readable storage medium to produce a particular machine, such that the particular machine becomes a means for implementing the functions specified herein. These program code instructions may also be stored in a computer-readable storage medium that can direct a computer, processing unit or other programmable apparatus to function in a particular manner to thereby generate a particular machine or particular article of manufacture. The instructions stored in the computer-readable storage medium may produce an article of manufacture, where the article of manufacture becomes a means for implementing functions described herein. The program code instructions may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions may produce a computer-implemented process such that the instructions executed by the computer, processing circuitry or other programmable apparatus provide operations for implementing functions described herein.

Execution of instructions by processing unit, or storage of instructions in a computer-readable storage medium, supports combinations of operations for performing the specified functions. In this manner, a computer system (1) may include processing unit (20) and a computer-readable storage medium or memory (50) coupled to the processing circuitry, where the processing circuitry is configured to execute computer-readable program code (60) stored in the memory. It will also be understood that one or more functions, and combinations of functions, may be implemented by special purpose hardware-based computer systems and/or processing circuitry which perform the specified functions, or combinations of special purpose hardware and program code instructions.

## Claims

1. A computer-implemented method, the method comprising:
- determining a candidate, wherein the candidate is related to a gene and a gene product of a pest organism,
- generating a gene representation, wherein the gene representation is a numerical representation of the gene,
- generating a product representation, wherein the product representation is a numerical representation of the gene product,
- generating an interrelation representation, wherein the interrelation representation is a numerical representation of interrelations of the gene and/or gene product with other genes and/or gene products of the organism,
- generating a gene expression response representation, wherein the gene expression response representation is a numerical representation of one or more responses of an expression level of the gene to one or more external stimuli,
- generating a candidate representation based on the gene representation, the product representation, the interrelation representation, and the gene expression response representation,
- providing a trained machine learning model, wherein the machine learning model is configured and was trained to determine how essential a gene and/or gene product is for an organism,
- inputting the candidate representation into the trained machine learning model,
- receiving information about how essential the gene and/or gene product is to the organism as an output from the trained machine learning model,
- outputting the information.

2. The method of claim 1, wherein the gene representation is or comprises a representation of a sequence of nucleotides.

3. The method of claim 1 or 2, wherein the gene representation is or comprises a predicted gene ontology annotation.

4. The method of any one of claims 1 to 3, wherein the gene representation is or comprises an embedding generated by a trained machine learning model based on gene data.

5. The method of any one of claims 1 to 4, wherein the gene representation is or comprises an essentiality score generated by a trained machine learning model based on gene data.

6. The method of any one of claims 1 to 5, wherein the product representation is or comprises a representation of an amino acid sequence of a protein or peptide encoded by the gene.

7. The method of any one of claims 1 to 6, wherein the product representation is or comprises a protein representation, wherein the protein representation comprises an embedding generated by a trained machine learning model based on protein data, wherein the protein data comprises an amino acid sequence and/or chemical and/or physical and/or biological property data of the protein encoded by the gene.

8. The method of claim 7, wherein the trained machine learning model for generating the protein embedding is or comprises a protein language model.

9. The method of any one of claims 1 to 8, wherein the interrelation representation is or comprises a gene co-expression representation.

10. The method of claim 9, wherein the gene co-expression representation is obtained from a gene co-expression network that maps relationships of the gene with other genes of the organism based on expression levels.

11. The method of any one of claims 1 to 10, wherein the interrelation representation comprises correlation coefficients between the gene and a number of nearest neighbour genes in a gene co-expression network, and/or a number of edges that connect the gene in a gene co-expression network with other genes, and/or an expression value of the gene.

12. The method of any one of claims 1 to 11, wherein the gene expression response representation represents one or more responses of the gene under treatment with one or more chemical compounds.

13. The method of claim 12, wherein the one or more chemical compounds comprise one or more active ingredients of one or more crop protection products,

14. The method of any one of claims 1 to 13, wherein the gene expression response representation comprises one or more parameters of a mathematical function, wherein the mathematical function expresses gene expression level as a function of an amount of a chemical compound and time.

15. The method of any one of claims 1 to 14, wherein the pest organism is a plant pest, such as an animal plant pest, a fungus, a bacterium, a virus or a weed.

16. A computer system comprising:
a processing unit; and
a memory storing a computer program configured, when executed by the processing unit, to cause the computer system to perform the method of any one of claims 1 to 15.

17. A non-transitory computer readable storage medium having stored thereon a computer program that, when executed by a processing unit of a computer system, cause the computer system to execute the method of any one of claims 1 to 15.

## Amended claims

### Amended claims in accordance with Rule 137(2) EPC.

1. A computer-implemented method, the method comprising:
- determining a candidate (C), wherein the candidate (C) is related to a gene (G^{C}) and a gene product (P^{C}) of a pest organism,
- generating a gene representation (GR^{C}),
o wherein the gene representation (GR^{C}) is a numerical representation of the gene (G^{C}),
o wherein the gene representation (GR^{C}) is or comprises a representation of a sequence of nucleotides,
- generating a product representation (PR^{C}),
o wherein the product representation (PR^{C}) is a numerical representation of the gene product (P^{C}),
o wherein the product representation (PR^{C}) is or comprises a representation of an amino acid sequence of a protein or peptide encoded by the gene (G^{C}),
- generating an interrelation representation (IR^{C}),
o wherein the interrelation representation (IR^{C}) is a numerical representation of interrelations of the gene (G^{C}) and/or gene product (P^{C}) with other genes and/or gene products of the organism,
o wherein the interrelation representation (IR^{C}) is or comprises a gene co-expression representation, wherein the gene co-expression representation is obtained from a gene co-expression network that maps relationships of the gene (G^{C}) with other genes of the organism based on expression levels,
o wherein the interrelation representation (IR^{C}) comprises correlation coefficients between the gene (G^{C}) and a number of nearest neighbour genes in the gene co-expression network, and/or a number of edges that connect the gene (G^{C}) in the gene co-expression network with other genes, and/or an expression value of the gene (G^{C}),
- generating a gene expression response representation (GER^{C}),
o wherein the gene expression response representation (GER^{C}) is a numerical representation of one or more responses of an expression level of the gene (G^{C}) to one or more external stimuli,
o wherein the gene expression response representation (GER^{C}) represents one or more responses of the gene (G^{C}) under treatment with one or more chemical compounds, wherein the one or more chemical compounds comprise one or more active ingredients of one or more crop protection products,
- generating a candidate representation (R^{C}) based on the gene representation (GR^{C}), the product representation (PR^{C}), the interrelation representation (IR^{C}), and the gene expression response representation (GER^{C}),
- providing a trained machine learning model (MLM^{t}),
o wherein the machine learning model (MLM^{t}) is configured and was trained to determine how essential a gene and/or gene product is for an organism,
o wherein the machine learning model (MLM^{t}) was trained based on training data, wherein the training data comprised, for each reference (R) of a plurality of references, (i) a gene representation (GR^{R}), a product representation (PR^{R}), an interrelation representation (IR^{R}), a gene expression response representation (GER^{R}) and optionally one or more further representations (FD^{R}), as input data and (ii) an information (E^{R}) about the essentiality of the reference (R) as target data, wherein each reference (R) relates to a gene (G^{R}) and corresponding gene product (P^{R}),
- inputting the candidate representation (R^{C}) into the trained machine learning model (MLM^{t}),
- receiving information (E^{C*}) about how essential the gene (G^{C}) and/or gene product (P^{C}) is to the organism as an output from the trained machine learning model (MLM^{t}),
- outputting the information (E^{C*}).

2. The method of claim 1, wherein the gene representation (GR^{C}) is or comprises a predicted gene ontology annotation.

3. The method of any one of claims 1 to 2, wherein the gene representation (GR^{C}) is or comprises an embedding generated by a trained machine learning model based on gene data.

4. The method of any one of claims 1 to 3, wherein the gene representation (GR^{C}) is or comprises an essentiality score generated by a trained machine learning model based on gene data.

5. The method of any one of claims 1 to 4, wherein the product representation (PR^{C}) is or comprises a protein representation, wherein the protein representation comprises an embedding generated by a trained machine learning model based on protein data, wherein the protein data comprises an amino acid sequence and/or chemical and/or physical and/or biological property data of the protein encoded by the gene.

6. The method of claim 5, wherein the trained machine learning model for generating the protein embedding is or comprises a protein language model.

7. The method of any one of claims 1 to 6, wherein the gene expression response representation (GER^{C}) comprises one or more parameters of a mathematical function, wherein the mathematical function expresses gene expression level as a function of an amount of a chemical compound and time.

8. The method of any one of claims 1 to 7, wherein the pest organism is a plant pest, such as an animal plant pest, a fungus, a bacterium, a virus or a weed.

9. A computer system (1) comprising:
a processing unit (20); and
a memory (50) storing a computer program (60) configured, when executed by the processing unit (20), to cause the computer system (1) to perform the method of any one of claims 1 to 8.

10. A non-transitory computer readable storage medium having stored thereon a computer program (60) that, when executed by a processing unit (20) of a computer system (1), cause the computer system (1) to execute the method of any one of claims 1 to 8.
